(19) Europäisches Patentamt
European Patent Office
Office européen des brevets

(11) Numéro de publication: **0 184 573**
**A1**

(12) **DEMANDE DE BREVET EUROPEEN**

(21) Numéro de dépôt: **85870166.7**

(22) Date de dépôt: **28.11.85**

(51) Int. Cl.[4]: **C 07 C 59/48**
C 07 C 59/64, C 07 C 51/00
C 07 D 333/38

(30) Priorité: **29.11.84 FR 8418202**

(43) Date de publication de la demande:
**11.06.86 Bulletin 86/24**

(84) Etats contractants désignés:
**AT BE CH DE FR GB IT LI LU NL SE**

(71) Demandeur: **SANOFI, société anonyme**
**40, Avenue George V**
**F-75008 Paris(FR)**

(71) Demandeur: **INDUSTRIA CHIMICA PRODUTTI FRANCIS S.p.A.**
**Via Origgio, 23**
**I-21042 Caronno P. LLa. (VA)(IT)**

(72) Inventeur: **Andre, Jean-Daniel**
**Square Horizon Bâtiment B**
**F-04200 Sisteron(FR)**

(72) Inventeur: **Heymes, Alain**
**Rue Frédéric Mistral, 5**
**F-04200 Sisteron(FR)**

(72) Inventeur: **Grossi, Pierre-Jean**
**Rue des Chapelles, 2**
**F-30390 Aramon(FR)**

(72) Inventeur: **Venerucci Manzaroli, Dott. Giovanni**
**Via Guerrazzi, 25**
**I-20145 Milano(IT)**

(74) Mandataire: **Cauchie, Daniel**
**c/o S.A. LABAZ-SANOFI N.V. Avenue De Béjar, 1**
**B-1120 Bruxelles(BE)**

(54) **Procédé de préparation d'acides alpha-hydroxy-alcanoiques et composés obtenus par ce procédé.**

(57) L'invention se rapporte à un procédé de préparation d'α-hydroxy-acides de formule générale :

```
      OH
      |
Cy-C-CO2H
      |
      R
```

dans laquelle R représente hydrogène ou un radical alkyle inférieur et Cy représente un radical phényl, naphtyle ou hétérocyclique, ce radical comportant éventuellement un ou plusieurs substituants sélectionnés du groupe formé par des radicaux alkyl inférieur, alkényle inférieur, alkynyle inférieur, alkoxy inférieur et par des atomes d'halogène, procédé suivant lequel :

– on traite une cétone α-monohalogénée de formule générale :

```
      O
      ||
Cy-C-CH-X
        |
        R
```

dans lequelle R et Cy ont la même signification que précédemment et X représente chlore, brome ou iode en présence d'une solution aqueuse d'un hydroxyde de métal alcalin, d'un solvant organique non polaire sélectionné d'un hydrocarbure aromatique ou alicyclique et d'un excès d'oxygène, accompagné éventuellement d'un gas inerte, et ce, à une température comprise entre la température d'ébullition du milieu réactionnel à pression atmosphérique et 240°C sous pression,

– puis en acidifie le sel de métal alcalin formé pour obtenir l'acide désiré.

EP 0 184 573 A1

TITRE MODIFIÉ
voir page de garde



PROCEDE DE PREPARATION D'α-HYDROXY-ACIDES ET COMPOSES OBTENUS PAR CE PROCEDE

La présente invention se rapporte, d'une manière générale, à un nouveau procédé de préparation d'α-hydroxy-acides ainsi qu'aux produits obtenus par ce procédé.

L'invention concerne notamment un nouveau procédé de préparation d'α-hydroxy-acides de formule générale :

$$\begin{array}{c} OH \\ | \\ Cy\text{-}C\text{-}CO_2H \\ | \\ R \end{array} \qquad I$$

dans laquelle R représente hydrogène ou un radical alkyle inférieur et Cy représente un radical phényle, naphtyle ou hétérocyclique, ce radical comportant éventuellement un ou plusieurs substituants sélectionnés du groupe formé par des radicaux alkyle inférieur, alkényle inférieur, alkynyle inférieur, alkoxy inférieur et par des atomes d'halogène tels que chlore ou brome.

Dans le présent contexte, les termes repris ci-dessus comportent la signification suivante :

"alkyle inférieur" désigne les restes d'hydrocarbures aliphatiques saturés ayant jusqu'à 4 atomes de carbone tels que méthyle, éthyle, n-propyle, isopropyle, n-butyle, isobutyle ou tertiobutyle;

"alkényle inférieur" désigne les restes d'hydrocarbures aliphatiques insaturés comportant une ou deux doubles liaisons et ayant de 2 à 4 atomes de carbone tels que vinyle, allyle ou butènyle;

"alkényle inférieur" désigne les restes d'hydrocarbures aliphatiques insaturés comportant une ou deux triples liaisons et ayant de 2 à 4 atomes de carbone tels qu'éthynyle, propargyle ou butynyle;

"alkoxy inférieur" désigne le groupement hydroxyle substitué par un radical alkyle inférieur tel que ci-dessus;

"radical hétérocyclique" désigne essentiellement un radical furyle, thiényle, pyrrolyle, benzofuryle, benzothiényle ou indolyle.

Ainsi, en tenant compte des valeurs données ci-dessus, le radical Cy peut représenter notamment un radical isobutyl-phényle de préférence isobutyl-4 phényle, un radical méthoxy-naphtyle de préférence méthoxy-6

naphtyle-2, un radical chloro-5 ou bromo-5 méthoxy-6 naphtyl-2 ou un radical thiényle de préférence thiényl-2.

Ces composés sont particulièrement utiles notamment comme intermédiaires dans la synthèse des acides alcanoïques de formule générale :

$$Cy-\underset{\displaystyle R}{\underset{|}{CH}}-\overset{\displaystyle O}{\overset{\|}{C}}OH \qquad (I')$$

dans laquelle R et Cy ont la même signification que précédemment.

Parmi ces composés qui sont connus notamment comme agents anti-inflammatoires, antipyrétiques ou antalgiques, on peut citer l'acide (isobutyl-4 phényl)-2 acétique ou ibufenac, l'acide (isobutyl-4 phényl)-2 propionique ou ibuprofène, l'acide (isobutyl-4 phényl)-2 butyrique ou butibufène, l'acide (méthoxy-6 naphtyl-2)-2 propionique ou naproxène ou encore l'acide (thiényl-2) -2 propionique.

On a décrit dans la demande de brevet japonais No. 54-39042 (C.A. <u>91</u>, P 140574u) la synthèse de l'ibuprofène à partir d'acide (isobutyl-4 phényl)-2 hydroxy-2 propionique ainsi que la préparation de cet intermédiaire.

Suivant le procédé cité, on prépare l'α-hydroxy-acide en question à partir d'(isobutyl-4 phényl)-2 oxo-2 acétate d'éthyle par réaction avec un halogénure de méthyl magnésium en milieu éther suivie d'une hydrolyse alcaline selon les conditions de la réaction de GRIGNARD.

Bien que les rendements obtenus soient importants, les conditions opératoires d'une telle réaction sont coûteuses et difficiles à mettre en oeuvre sur le plan industriel (utilisation du magnésium, milieu réactionnel anhydre etc...). D'autres publications rapportent également des procédés de préparation des acides α-hydroxy-alcanoïques de formule (I').

Dans la plupart des cas, ces procédés comportent des inconvénients les écartant d'une mise en oeuvre industrielle, inconvénients qui peuvent provenir par exemple de l'utilisation de produits de départ relativement difficiles à obtenir.

On connaît cependant un procédé de préparation de certains acides α-hydroxy-phénylalcanoïques faisant intervenir des conditions opératoires extrapolables sans difficultés majeures sur le plan industriel.

Ce procédé, qui a été décrit notamment dans J.A.C.S. 72, 1642-1644 (1950) ou dans Org.Synth. III, 538-541 (1955) repose sur une réaction de transposition de phénylalkylcétones α,α-dihalogénées faisant intervenir l'hydroxyde de sodium aqueux.

On a tenté de préparer des acides de formule I et en particulier l'acide (isobutyl-4 phényl)-2 hydroxy-2 propionique suivant une telle méthode mais avec un succès médiocre.

Les rendements maximum obtenus n'ont été que de 19% environ en acide désiré, l'acide isobutyl-4 benzoïque étant le produit préférentiellement synthétisé. Par conséquent, l'obtention des acides α-hydroxy-phénylalcanoïques de formule I suivant un procédé exploitable industriellement reste d'un intérêt primordial.

On a maintenant trouvé, suivant la présente invention, qu'il est possible de préparer les α-hydroxy-acides de formule I, selon un tel procédé industriel, au moyen d'une réaction de transposition non pas d'une cétone α,α-dihalogénée mais d'une cétone α-monohalogénée en faisant intervenir l'oxygène, un hydroxyde de métal alcalin en solution aqueuse et un solvant organique non polaire.

Ainsi, le procédé de l'invention pour la préparation des acides α-hydroxy-alcanoïques en question consiste :

- à traiter une cétone α-monohalogénée de formule générale :

$$Cy-\overset{\overset{O}{\|}}{C}-\underset{\underset{R}{|}}{CH}-X \qquad II$$

dans laquelle R et Cy ont la même signification que précédemment et X représente chlore, brome ou iode en présence d'une solution aqueuse d'un hydroxyde de métal alcalin tel que l'hydroxyde de lithium, de sodium ou de potassium, d'un solvant organique non polaire sélectionné d'un hydrocarbure aromatique ou alicyclique et d'un excès d'oxygène, accompagné éventuellement d'un gaz inerte formant un mélange tel que l'air, et ce, à une température comprise entre la température d'ébullition du milieu réactionnel à pression atmosphérique et 240°C sous pression.

- puis à acidifier le sel de métal alcalin ainsi formé pour obtenir l'acide désiré.

Dans le cas particulier de l'acide (isobutyl-4 phényl)-2 hydroxy-2 propionique, le procédé de l'invention permet d'obtenir des rendements de l'ordre de 70 à 90% tout en limitant la formation de dérivés d'acide benzoïque produits par une réaction secondaire.

Comme hydrocarbure aromatique, on peut utiliser par exemple le toluène, les xylènes, l'isobutylbenzène ou le naphtalène et comme hydrocarbure alicyclique, par exemple le cyclohexane.

On préfère généralement, les xylènes, et en particulier le xylène industriel pour des raisons économiques.

On a remarqué que la présence d'oxygène en quantité au moins stoechiométrique au sein du milieu réactionnel s'avère indispensable pour la production des composés de formule I avec des rendements importants.

En effet, sous atmosphère inerte, les rendements en α-hydroxy-acide de formule I par exemple en acide (isobutyl-4 phényl)-2 hydroxy-2 propionique, se révèlent pratiquement nuls.

Dans le cas de la mise en oeuvre des cétones α-monohalogénées de formule II à la température d'ébullition du milieu réactionnel sous pression atmosphérique, on peut utiliser indifféremment l'oxygène pur ou l'oxygène accompagné d'un gaz inerte.

Toutefois, aux températures plus élevées, par exemple à des températures de l'ordre de 200°C, on préfère utiliser l'oxygène accompagné d'un gaz inerte. A la pression atmosphérique et à la température d'ébullition du mélange réactionnel, la réaction de transposition s'effectuera en 20 à 25 heures avec des rendements d'au moins 70%.

Sans être limitatives, les conditions opératoires suivantes seront généralement utilisées en présence d'un excès d'oxygène ou d'air :

0,5 à 40 parties en poids d'hydroxyde de métal alcalin

5 à 400 parties en volume d'eau

2 à 40 parties en volume de solvant organique

et ce, pour 1 partie en poids de cétone de formule II, la réaction ayant lieu à la température d'ébullition du mélange formé.

On a trouvé, en particulier, que la réaction de transposition selon l'invention peut être considérablement accélérée par élévation de la température. On a, en effet remarqué, qu'une élévation de la température

réactionnelle, de préférence entre 160 et 240°C, nécessitant des conditions opératoires sous pression, produit le même effet qu'une augmentation de la durée de la réaction.

Ainsi, à une température de 180 à 220°C, la réaction de transposition au sein du milieu réactionnel maintenu sous agitation et sous pression, par exemple par l'utilisation d'un autoclave agité par balancement, peut être accomplie en quelques minutes par exemple en 15 à 60 minutes.

A ces températures comprises entre la température d'ébullition du milieu réactionnel à pression atmosphérique et 240°C sous pression, les conditions opératoires suivantes en présence d'un excès d'air seront généralement utilisées sans pour autant constituer une limitation au procédé de l'invention :

0,5 à 10 parties en poids d'hydroxyde de métal alcalin
5 à 150 parties en volume d'eau
1 à 30 parties en volume de solvant organique

et ce, pour 1 partie en poids de cétone de formule II.

Selon un premier mode de mise en oeuvre du procédé de l'invention à une température comprise entre la température d'ébullition du milieu réactionnel à pression atmosphérique et 240°C sous pression, on mélange dans l'autoclave, l'hydroxyde de métal alcalin en solution, le xylène et la cétone monohalogénée de formule II, on chauffe l'autoclave fermé à la température désirée et on applique une pression d'oxygène éventuellement en mélange avec un gaz inerte, généralement une pression de l'ordre de 20 bars.

On peut également pratiquer selon un second mode opératoire qui consiste à :

- charger au départ dans l'autoclave la solution aqueuse d'hydroxyde de métal alcalin ainsi que le xylène si la cétone monohalogénée de formule II est liquide,
- chauffer l'autoclave fermé jusqu'à l'obtention de la température désirée, puis à introduire l'oxygène ou l'oxygène en mélange avec un gaz inerte ensuite, à l'aide d'une pompe doseuse, la cétone monohalogénée de formule II telle quelle ou en solution dans le xylène.
  Généralement, l'introduction de la cétone monohalogénée s'effectue en 2h environ.

Cette dernière façon d'opérer, par addition en continu de la cétone

monohalogénée permet d'accroître considérablement la productivité en α-hydroxy-acide de formule I.

La séparation et la purification des α-hydroxy-acides de formule I peut être obtenue par précipitation de leurs sels métalliques à partir du mélange réactionnel puis acidification. Cette mise en oeuvre évite la nécessité d'acidifier la totalité du mélange contenant un excès de base et l'obligation d'extraire les α-hydroxy-acides en question au sein d'un volume d'eau important.

De plus, un avantage non négligeable provient du fait qu'au cours de la précipitation des sels métalliques des α-hydroxy-acides, les autres impuretés acides de même que les halogénures de sodium formés ne sont pas entraînés dans le précipité et demeurent presque totalement en raison de leurs plus grandes solubilités aussi bien dans le milieu réactionnel que dans l'eau. Ces sels d'α-hydroxy-acides peuvent donc être éliminés par filtration. Cet avantage est d'autant plus intéressant car les réactions ultérieures conduisant aux acides alcanoïques de formule (I') ne produisent pas ou peu d'impuretés. On pourra donc obtenir ces acides alcanoïques purs par purification aisée des produits de départ en l'occurrence les acides α-hydroxy-alcanoïques de formule I alors que selon d'autres procédés antérieurs la purification ne peut se faire qu'au niveau de l'acide alcanoïque, purification qui se révèle difficile à ce stade.

Un autre avantage du procédé de l'invention se situe au niveau de la mise en oeuvre des composés de départ particulièrement intéressants, à savoir les composés de formule II, en raison de la facilité avec laquelle ils peuvent être produits.

Ces composés de formule II peuvent être obtenus, par exemple, à partir d'un composé de formule générale :

$$Cy\text{-}H \qquad III$$

dans laquelle Cy a la même valeur que précédemment, par acylation selon les conditions de la réaction de FRIEDEL-CRAFTS, au moyen des halogénures d'acyle α-monohalogénés de formule générale :

$$Y\text{-}\overset{\overset{O}{\|}}{C}\text{-}\underset{\underset{R}{|}}{CH}\text{-}X \qquad IV$$

dans laquelle R et X ont la même signification que précédemment et Y représente chlore ou brome, ou au moyen des anhydrides ou autres dérivés équivalents des composés de formule IV ci-dessus.

D'autres procédés peuvent être utilisés pour l'obtention des composés de formule II.

Selon une variante, on peut préparer ces composés à partir d'une cétone de formule générale :

$$Cy-\overset{O}{\overset{\|}{C}}-CH_2R \qquad V$$

dans laquelle R et Cy ont la même signification que précédemment, soit par chloration au moyen de chlore dans le chlorure de méthylène à 20°C ou dans le N,N-diméthylformamide à 90-95°C ou au moyen de chlorure cuivrique dans le N,N-diméthylformamide à 80-90°C selon la méthode décrite dans J. Org. Chem. 28, 630-633 (1963) soit par l'action du brome à 20°C dans un solvant approprié par exemple le dioxanne ou un mélange éther éthylique/dioxanne selon la méthode décrite dans C.A. 97, 162599g (1982) ou encore au moyen de tribromure de pyridinium dans le dioxanne.

Un excès de chlore dans le chlorure de méthylène à 20°C ou dans le N,N-diméthylformamide à 90-95°C ou un excès de tribromure de pyridinium dans le dioxanne permet, dans certains cas, de provoquer,outre la formation d'une α-monohalogéno-cétone, la substitution du cycle Cy par respectivement un atome de chlore ou de brome.

Par exemple le traitement de la (méthoxy-6 naphtyl-2)-1 propanone-1 par un excès d'agent de chloration ou de bromation tel qu'indiqué précédemment provoque la substitution du cycle naphtyle en position 5 par respectivement un atome de chlore ou de brome et la formation de chloro-2 (chloro-5 méthoxy-6 naphtyl-2)-1 propanone-1 (rendement : 87%) et de bromo-2 (bromo-5 méthoxy-6 naphtyl-2)-1 propanone-1.

Comme indiqué précédemment, les acides α-hydroxy-alcanoïques de formule I peuvent être utilisés comme intermédiaires de synthèse pour la préparation des acides alcanoïques de formule (I') ci-dessus.

A cet effet, les composés de formule I pourront être mis en oeuvre suivant des procédures telles que :

a) déshydratation au reflux dans un solvant au moyen, par exemple d'acide p-toluènesulfonique suivie d'hydrogénation catalytique dans un solvant de l'acide alcène-2 oïque obtenu, par application de la méthode décrite dans la demande de brevet de la République Fédérale d'Allemagne No. 2.613.817.

b) hydrogénolyse des α-hydroxy-acides par exemple en présence de nickel de RANEY dans l'acide acétique à 170°C et sous 16 atmosphères tel que décrit dans la demande de brevet japonais No. 53-02449 (CA, 89, 6118d) ou en présence de charbon palladié dans l'acide acétique à 60°C et sous 30 atmosphères comme décrit dans la demande de brevet japonais No. 53-34745 (CA, 89, 108684e).

Il est également possible de modifier les méthodes antérieures ci-dessus en utilisant soit le charbon palladié et l'acide sulfurique comme catalyseur, ce qui permet d'opérer à pression atmosphérique, soit l'acide iodhydrique dans l'acide acétique.

Comme mentionné précédemment, le procédé de l'invention s'est révélé de loin supérieur aux procédés suggérés par l'état de la technique ou interviennent des réactions de transposition de cétones α,α-dihalogénées.

A cet effet, on a pratiqué des essais de transposition de la bromo-2 (isobutyl-4 phényl)-1 propanone-1 en acide (isobutyl-4 phényl)-2 hydroxy-2 propionique suivant le procédé de l'invention.

On a obtenu les résultats suivants :

| Réactifs et solvant pour 1g de cétone | | | Réaction de transposition | | Rendements molaires (%) | |
|---|---|---|---|---|---|---|
| NaOH (g) | Eau (ml) | Xylène (ml) | Température | Durée (h) | A* | B** |
| 20 | 400 | 40 | Ebullition | 24h dont 7h avec barbotage d'air | 78 | 27 |
| 20 | 200 | 40 | Ebullition | 24h avec barbotage d'oxygène | 79 | 18 |

A titre de comparaison, on a pratiqué des essais de transposition d'une phénylalkylcétone α,α-dihalogénée à savoir la dichloro-2,2 (isobutyl)-4

phényl)-1 propanone-1 en acide (isobutyl-4 phényl)-2 hydroxy-2 propionique selon des conditions de l'art antérieur.

| Conditions opératoires | Rendements molaires (%) | |
|---|---|---|
| | A* | B** |
| NaOH 20%/eau/20°C/29 h | 0 | 7 |
| NaOH 20%/eau/60°C/2,5 h + éthanol/1h supplémentaire | 0 | 33 |
| NaOH 32%/eau/20°C/6h + éthylèneglycol + éthanol 1h | 19 | 40 |

* acide (isobutyl-4 phényl)-2 hydroxy-2 propionique

** acide isobutyl-4 benzoïque

Ces résultats montrent la très nette supériorité du procédé de l'invention.

Les Exemples, non limitatifs suivants, illustrent le procédé de l'invention :

EXEMPLE 1

Préparation de l'acide (isobutyl-4 phényl)-2 hydroxy-2 propionique

a) Bromo-2 (isobutyl-4 phényl)-1 propanone-1

Sous agitation, on ajoute, en 50 min. et à 20-25°C, 163g (1,02 mole) de brome à une solution de 184g (0,97 mole) d'(isobutyl-4 phényl)-1 propanone-1 dans 150ml d'éther éthylique anhydre et 37ml de dioxanne anhydre. Le milieu reste coloré en fin d'introduction.

On agite encore durant 30 min. à 20-25°C puis on ajoute 370ml d'eau sans dépasser 25°C. Après extraction à l'éther éthylique, on lave les phases organiques à l'eau salée, sèche sur sulfate de sodium et amène à sec sous vide. On obtient ainsi 272g de bromo-2 (isobutyl-4 phényl)-1 propanone-1 titrant 89% en chromatographie phase gazeuse (C.P.G.) soit un rendement molaire de 92,9%.

On prépare un échantillon analytique par cristallisation dans le méthanol.

P.F. : 61,4°C

C.P.G. : 99%

I.R. (film)

1685, 1610 $cm^{-1}$

R.M.N. ($CCl_4$)

7,90 et 7,20 (2d, 4H) ; 5,20 (q, 1H) ; 2,55 (d, 2H) ; 1,85 (d et m, 4H) ; 0,95 ppm (d, 6H)

Analyse

| | | | | | | |
|---|---|---|---|---|---|---|
| C | calculé | : | 58,00% | trouvé | : | 57,95% |
| H | calculé | : | 6,36% | trouvé | : | 6,42% |
| Br | calculé | : | 29,69% | trouvé | : | 30,10% |

b) Acide (isobutyl-4 phényl)-2 hydroxy-2 propionique

Dans un autoclave de 500ml, on introduit 2g ($7,3.10^{-3}$ mole) de bromo-2 (isobutyl-4 phényl)-1 propanone-1 (C.P.G. : 98,2%), 7,2g (0,18 mole) d'hydroxyde de sodium en pastilles, 200ml d'eau et 40ml de xylène. On porte le mélange réactionnel à 200°C puis on applique une pression d'air de 20 bars en agitant pendant 15 min. dans ces conditions. Après refroidissement à 20°C et dépressurisation, on décante et réextrait les phases aqueuses à l'éther éthylique. Après acidification à pH=1 avec de l'acide chlorhydrique concentré, on extrait à nouveau à l'éther éthylique. On lave à l'eau ces dernières phases éthérées, sèche sur sulfate de sodium et amène à sec sous vide.

On obtient ainsi 1,45g d'acide (isobutyl-4 phényl)-2 hydroxy-2 propionique titrant 85,2% en C.P.G. soit un rendement de 76,2%.

En opérant de la même manière que précédemment mais en apportant les modifications ci-dessous, on a obtenu l'acide (isobutyl-4 phényl)-2 hydroxy-2 propionique avec les rendements suivants :

a) mélange réactionnel à 200°C avec application d'une pression d'air de 20 bars durant 1 heure.
Rendement : 77%

b) mélange réactionnel à 220°C avec application d'une pression d'air de 28 bars durant 30 minutes.
Rendement : 72%.

EXEMPLE 2

Préparation de l'acide (isobutyl-4 phényl)-2 hydroxy-2 propionique

a) Chloro-2 (isobutyl-4 phényl)-1 propanone-1

On dissout 40,9g (0,24 mole) de chlorure cuivrique dihydraté et 5,09g (0,12 mole) de chlorure de lithium dans 60ml de diméthylformamide. La dissolution est exothermique et la température s'élève à 50°C. On chauffe vers 80°C et on ajoute, en 5 min. 19g (0,1 mole) d'(isobutyl-4 phényl)-1 propanone-1.

On agite le milieu réactionnel durant 6 heures à 80-90°C puis on refroidit à 20°C. On verse le mélange dans de l'eau glacée et on acidifie à pH=1 avec de l'acide chlorhydrique concentré. Après extraction à l'éther éthylique, on lave les phases organiques, à l'eau acidulée puis à l'eau. On sèche sur sulfate de sodium et on amène à sec sous vide.

On obtient ainsi 22,3g de chloro-2 (isobutyl-4 phényl)-1 propanone-1 titrant 90,6% en C.P.G. soit un rendement de 89,9%.

On prépare un échantillon analytique par cristallisation dans le méthanol.

P.F. : 50°C

C.P.G. : 99,6%

I.R. (film)

1690, 1610 $cm^{-1}$

R.M.N. ($CCl_4$)

7,85 et 7,15 (2d, 4H) ; 5,10 (q, 1H) ; 2,50 (d, 2H) ; 2,00 (m, 1H) ; 1,65 (d, 3H) ; 0,90 ppm (d, 6H)

Analyse

| | | | | | |
|---|---|---|---|---|---|
| C | calculé | : 69,48% | trouvé | : | 69,64% |
| H | calculé | : 7,62% | trouvé | : | 7,31% |
| Cl | calculé | : 15,78% | trouvé | : | 15,78% |

b) Acide (isobutyl-4 phényl)-2 hydroxy-2 propionique

En opérant comme décrit dans l'Exemple 1 à partir de chloro-2 (isobutyl-4 phényl)-1 propanone-1 (C.P.G. : 94,7%), on obtient 1,75g d'acide (isobutyl-4 phényl)-2 hydroxy-2 propionique titrant 78,6% en C.P.G. soit un rendement de 74%.

EXEMPLE 3

Préparation de l'acide (isobutyl-4 phényl)-2 hydroxy-2 acétique

a) Bromo-2 (isobutyl-4 phényl)-1 éthanone

En 15 min. et à 20-25°C, on ajoute 33,7g (0,21 mole) de chlorure de bromacétyle à une suspension de 28g (0,21 mole) de chlorure d'aluminium dans 80ml de chlorure de méthylène anhydre.

On obtient une suspension beige à laquelle on ajoute 26,9g (0,2 mole) d'isobutylbenzène en 10 min. à 20-25°C. On agite le milieu réactionnel durant 4h30 min. à 20-25°C puis on introduit 100ml d'acide chlorhydrique à 10%

sans dépasser 25°C en refroidissant au moyen d'un bain d'eau glacée. On décante et extrait la phase aqueuse avec du chlorure de méthylène. On lave les phases organiques à l'eau, sèche sur sulfate de sodium et amène à sec sous vide.

On obtient ainsi 50,1g de bromo-2 (isobutyl-4 phényl)-1 éthanone titrant 92,2% en C.P.G. soit un rendement de 90,4%.
On prépare un échantillon analytique par cristallisation dans l'hexane.
P.F. : 39°C
C.P.G. : 97,8%
I.R. (KBr)
1680, 1610 $cm^{-1}$
R.M.N. ($CDCl_3$)
7,80 et 7,15 (2d, 4H) ; 4,35 (s, 2H) ; 2,45 (d, 2H) ; 1,90 (m, 1H) ; 0,90 ppm (d, 6H)
Analyse

| | | | | | | |
|---|---|---|---|---|---|---|
| C | calculé | : | 56,53% | trouvé | : | 56,93% |
| H | calculé | : | 5,93% | trouvé | : | 6,03% |
| Br | calculé | : | 31,26% | trouvé | : | 31,00% |

En opérant comme décrit ci-dessus avec le chlorure de chloroacétyle, on obtient 39,9g de chloro-2 (isobutyl-4 phényl)-1 éthanone titrant 95,5% en C.P.G. soit un rendement de 90,5%.
On obtient un échantillon analytique par cristallisation dans l'hexane.
P.F. : 33,4°C
C.P.G. : 97,5%
I.R. (KBr)
1710, 1690, 1610 $cm^{-1}$
R.M.N. ($CDCl_3$)
7,90 et 7,30 (2d, 4H) : 4,70 (s, 2H) ; 2,55 (d, 2H) ; 1,90 (m, 1H) ; 0,95 ppm (d, 6H)
Analyse

| | | | | | | |
|---|---|---|---|---|---|---|
| C | calculé | : | 68,41% | trouvé | : | 68,08% |
| H | calculé | : | 7,18% | trouvé | : | 7,17% |
| Br | calculé | : | 16,83% | trouvé | : | 16,73% |

b) Acide (isobutyl-4 phényl)-2 hydroxy-2 acétique

1) En opérant comme décrit dans l'Exemple 1, à partir de bromo-2 (isobutyl-4

phényl)-1 éthanone (C.P.G. : 96,2%), on obtient 1,44g d'acide (isobutyl-4 phényl)-2 hydroxy-2 acétique titrant 75,6% en C.P.G. soit un rendement de 69,5%.

2) En opérant comme décrit dans l'Exemple 1, à partir de chloro-2 (isobutyl-4 phényl)-1 éthanone (C.P.G. : 98,2%), on obtient 1,59g d'acide (isobutyl-4 phényl)-2 hydroxy-2 acétique titrant 80% en C.P.G. soit un rendement de 65,7%.

## EXEMPLE 4

### Préparation de l'acide (isobutyl-4 phényl)-2 hydroxy-2 butanoïque

a) Bromo-2 (isobutyl-4 phényl)-1 butanone-1

En opérant comme décrit dans l'Exemple 1 à partir d'(isobutyl-4 phényl) -1 butanone-1, on obtient 14g de bromo-2 (isobutyl-4 phényl)-1 butanone-1 titrant 94,6% en C.P.G. soit un rendement de 95,7%.

On obtient après distillation sous vide :

C.P.G. : 94,4%

P.E. : 157°C sous 3 mm Hg

I.R. (film)

1680, 1605 $cm^{-1}$

R.M.N. ($CDCl_3$)

7,90 et 7,20 (2d, 4H) ; 5,05 (t, 1H) ; 2,50 (d, 2H) ; 2,10 (m, 3H) ; 1,05 et 0,90 ppm (t et d, 9H)

$n_D^{22}$ : 1,5425

Analyse

| | | | | |
|---|---|---|---|---|
| C | calculé | : 59,37% | trouvé | : 59,04% |
| H | calculé | : 6,76% | trouvé | : 6,92% |
| Br | calculé | : 28,21% | trouvé | : 28,26% |

b) Acide (isobutyl-4 phényl)-2 hydroxy-2 butanoïque

En opérant comme décrit dans l'Exemple 1 à partir de bromo-2 (isobutyl-4 phényl)-1 butanone-1 (C.P.G. : 93,5%), on obtient 1,34g d'acide (isobutyl-4 phényl)-2 hydroxy-2 butanoïque titrant 60,9% en C.P.G. soit un rendement de 52,6%.

## EXEMPLE 5

### Préparation de l'acide (isobutyl-4 phényl)-2 hydroxy-2 butanoïque

a) Chloro-2 (isobutyl-4 phényl)-1 butanone-1

En opérant comme décrit dans l'Exemple 2 à partir d'(isobutyl-4 phényl) -1 butanone-1, on obtient 10,9g de chloro-2 (isobutyl-4 phényl)-1 butanone-1 titrant 81,8% en C.P.G. soit un rendement de 80,2%.

On obtient un échantillon purifié par chromatographie sur colonne de silice avec comme éluant l'hexane seul puis l'hexane contenant jusqu'à 2% d'acétate d'éthyle.

C.P.G. : 96,3%

P.E. : 111°C sous 1 mm Hg

I.R. (film)

1690, 1610 $cm^{-1}$

R.M.N. ($CDCl_3$)

7,90 et 7,25 (2d, 4H) ; 5,05 (t, 1H) ; 2,50 (d, 2H) ; 2,00 (m, 3H) ; 1,05 et 0,90 ppm (t, d, 9H).

$n_D^{22}$ : 1,5428

Analyse

| | | | | | | |
|---|---|---|---|---|---|---|
| C | calculé | : | 70,43% | trouvé | : | 70,77% |
| H | calculé | : | 8,02% | trouvé | : | 8,28% |
| Cl | calculé | : | 14,85% | trouvé | : | 14,72% |

b) Acide (isobutyl-4 phényl)-2 hydroxy-2 butanoïque

En opérant comme décrit dans l'Exemple 1 à partir de chloro-2 (isobutyl-4 phényl)-1 butanone-1 (C.P.G. : 93,1%), on obtient 1,32g d'acide (isobutyl-4 phényl)-2 hydroxy-2 butanoïque titrant 71,1% en C.P.G. soit un rendement de 51%.

EXEMPLE 6

Préparation de l'acide (isobutyl-4 phényl)-2 hydroxy-2 propionique.

Dans un ballon, on introduit 1g de bromo-2 (isobutyl-4 phényl)-1 propanone-1, 40g d'hydroxyde de sodium, 400ml d'eau et 40ml de xylène.

On porte le mélange à ébullition et on l'y maintient sous agitation, durant 24 heures dont 7 heures avec barbotage d'air.

Après avoir refroidi à 20°C, on décante et extrait les phases aqueuses à l'éther éthylique. Après acidification à pH=1 avec de l'acide chlorhydrique concentré, on réextrait à nouveau à l'éther éthylique.

On lave ces dernières phases éthérées à l'eau, on sèche sur sulfate de sodium

et on amène à sec sous vide.

On obtient ainsi 0,82g d'acide (isobutyl-4 phényl)-2 hydroxy-2 propionique titrant 78% en C.P.G. soit un rendement de 78%.

En opérant de la même manière que précédemment mais en maintenant le mélange réactionnel à l'ébullition durant 24h avec barbotage d'oxygène, on obtient 0,77g d'acide (isobutyl-4 phényl)-2 hydroxy-2 propionique titrant 84% en C.P.G. soit un rendement de 79%.

EXEMPLE 7

Préparation de l'acide (isobutyl-4 phényl)-2 hydroxy-2 propionique

Dans un autoclave de 2l, en acier inoxydable, équipé d'un agitateur central mécanique, on charge 300ml d'eau et 28,8g (0,72 mole) d'hydroxyde de sodium en pastilles. On ferme l'appareil et on porte la température interne à 180°C.

On branche alors une arrivée d'air sous pression de 20 bars. Sous agitation et en 2 heures, on introduit alors, au moyen d'une pompe, une solution de 40g (0,146 mole) de bromo-2 (isobutyl-4 phényl)-1 propanone-1 (C.P.G. : 98,24%) dans 80ml de xylène industriel. On maintient l'agitation et la température durant 15 minutes après la fin de l'introduction puis on refroidit l'appareil dans un courant d'air.

On ajoute à la masse réactionnelle refroidie à 20°C, 67g de chlorure de sodium, agite durant une heure et filtre sur verre fritté. On lave le précipité avec de l'acétate d'éthyle puis on reprend dans 360ml d'eau. On acidifie la suspension obtenue avec 13,4 ml d'acide chlorhydrique concentré puis on filtre. Après lavage à l'eau du précipité obtenu et sèchage à 50°C sous 5 mm Hg, on isole 25,5g d'acide (isobutyl-4 phényl)-2 hydroxy-2 propionique titrant 95,5% en C.P.G. soit un rendement molaire de 75,2%.

EXEMPLE 8

Préparation de l'acide (isobutyl-4 phényl)-2 hydroxy-2 propionique.

a) Chloro-2 (isobutyl-4 phényl)-1 propanone-1

On fait barboter du chlore pendant environ 6h à 20°C, dans une solution de 150g (0,75 mole) d'(isobutyl-4 phényl)-1 propanone-1 (C.P.G. : 94,7%) dans 900 ml de chlorure de méthylène anhydre.

On lave ensuite le milieu réactionnel à l'eau, sèche sur sulfate de sodium et amène à sec sous vide.

On obtient ainsi 173,7g de chloro-2 (isobutyl-4 phényl)-1 propanone-1 titrant 91,5% en C.P.G. soit un rendement de 94,8%.

b) Acide (isobutyl-4 phényl)-2 hydroxy-2 propionique

En opérant comme décrit dans l'Exemple 7, à partir de 34,2g de chloro-2 (isobutyl-4 phényl)-1 propanone (C.P.G. : 91,5%), on obtient 22,4g d'acide (isobutyl-4 phényl)-2 hydroxy-2 propionique titrant 98% en C.P.G., soit un rendement de 68,1%.

EXEMPLE 9

Préparation de l'acide hydroxy-2 (méthoxy-6 naphtyl-2)-2 propanone-1

a) Bromo-2 (méthoxy-6 naphtyl-2)-1 propanone-1

Sous agitation, on ajoute en 10 minutes et à 25°C, 26,88g ($84.10^{-3}$ mole) de tribromure de pyridinium (P.F. : 132°C) à une solution de 17,22g ($80.10^{-3}$ mole) de (méthoxy-6 naphtyl-2)-1 propanone-1 dans 120 ml de dioxanne anhydre.

On agite le milieu réactionnel durant 2h30 min à 25°C puis on additionne 400ml d'une solution à 3% de bisulfite de sodium en 15 min. environ.

On observe la formation d'une huile qui cristallise. On filtre le produit, on le lave à l'eau puis on le sèche.

On obtient ainsi 23g de bromo-2 (méthoxy-6 naphtyl-2)-1 propanone-1 titrant 88,6% en C.P.G. soit un rendement molaire de 86,9%.

On prépare un échantillon analytique par recristallisation dans l'éthanol

P.F. : 86,1°C

C.P.G. : 96,7%

I.R. (KBr)

1675, 1660, 1620 $cm^{-1}$

R.M.N. ($CDCl_3$)

8,50-7,00 (m, 6H) ; 5,40 (q, 1H) ; 3,90 (s, 3H) ; 1,90 ppm (d, 3H)

Analyse

| | | | | | |
|---|---|---|---|---|---|
| C | calculé | : 57,35% | trouvé | : | 56,93% |
| H | calculé | : 4,47% | trouvé | : | 4,38% |
| Br | calculé | : 27,26% | trouvé | : | 27,40% |

b) Acide hydroxy-2 (méthoxy-6 naphtyl-2)-2 propanoïque

Dans un autoclave de 2l en acier inoxydable, équipé d'un agitateur mécanique, on introduit 250ml d'eau, 9g ($225.10^{-3}$ mole) d'hydroxyde de sodium en pastilles, 2,5g ($8,2.10^{-3}$ mole) de bromo-2 (méthoxy-6 naphtyl-2)-1 propanone-1 et 50 ml de xylène.

On ferme l'appareil et on porte la température interne à 200°C. On branche alors une arrivée d'air sous pression de 20 bars et on agite durant 15 min. dans ces conditions puis on refroidit l'appareil dans un courant d'air. On extrait à l'éther éthylique. On acidifie la phase aqueuse jusqu'à pH=1 avec de l'acide chlorhydrique concentré et on extrait à nouveau à l'éther éthylique. On lave à l'eau ces dernières phases organiques, on les sèche sur sulfate de sodium et on les amène à sec sous vide.

On obtient ainsi 1,77g d'acide hydroxy-2 (méthoxy-6 naphtyl-2)-2 propionique titrant 75,7% en C.P.G., soit un rendement molaire de 66,4%.

On prépare un échantillon analytique par recristallisation dans un mélange chlorure de méthylène/éthanol.

<u>P.F.</u> : 173°C

<u>C.P.G.</u> : 99,1%

<u>I.R.</u> (KBr)

3420, 3200-2500, 1735, 1625-1600 $cm^{-1}$

<u>R.M.N.</u> ($CDCl_3/DMSOd_6$)

8,00-7,00 (m, 8H) ; 3,80 (s, 3H) ; 1,85 ppm (s, 3H)

<u>Analyse</u>

| | | | | | | |
|---|---|---|---|---|---|---|
| C | calculé | : | 68,28% | trouvé | : | 67,78% |
| H | calculé | : | 5,73% | trouvé | : | 5,67% |

## EXEMPLE 10

### Préparation de l'acide hydroxy-2 (méthoxy-6 naphtyl-2)-2 propionique

a) Chloro-2 (méthoxy-6 naphtyl-2)-1 propanone-1

On ajoute rapidement 8g ($37.10^{-3}$ mole) de (méthoxy-6 naphtyl-2)-1 propanone-1 à une solution de 15,14g ($88.10^{-3}$ mole) de chlorure cuivrique dihydraté et de 1,88g ($44.10^{-3}$ mole) de chlorure de lithium dans 35ml de diméthylformamide anhydre à 80°C. On agite le milieu réactionnel pendant 2 heures à 80-90°C puis on le verse sur de la glace pilée et on l'acidifie à pH=1 avec de l'acide chlorhydrique concentré. Après extraction à l'éther éthylique, on lave les phases organiques à l'eau salée, on les sèche sur sulfate de sodium et on les amène à sec sous vide.

On obtient ainsi 9,37g de chloro-2 (méthoxy-6 naphtyl-2)-1 propanone-1 titrant 92,4% en C.P.G. soit un rendement molaire de 94,2%.

On prépare un échantillon analytique par cristallisation dans un mélange hexane/éther éthylique.

P.F. : 74°C

C.P.G. : 96,9%

I.R. (KBr) : 1675, 1615 $cm^{-1}$

R.M.N. ($CDCl_3$)

8,50-7,00 (m, 6H) ; 5,30 (q, 1H) ; 3,9 (s, 3H) ; 1,75 ppm (d, 3H)

Analyse

| | | | | | | |
|---|---|---|---|---|---|---|
| C | calculé | : | 67,61% | trouvé | : | 66,83% |
| H | calculé | : | 5,27% | trouvé | : | 5,19% |
| Cl | calculé | : | 14,25% | trouvé | : | 13,95% |

b) Acide hydroxy-2 (méthoxy-6 naphtyl-2)-2 propionique

En opérant comme décrit dans l'Exemple 9 à partir de 2g ($7,6.10^{-3}$ mole) de chloro-2 (méthoxy-6 naphtyl-2)-1 propanone-1, on obtient 1,33g d'acide hydroxy-2 (méthoxy-6 naphtyl-2)-2 propionique titrant 77,5% en C.P. G. soit un rendement molaire de 55,1%.

EXEMPLE 11

Préparation de l'acide hydroxy-2 (thiényl-2)-2 propionique

a) Bromo-2 (thiényl-2)-1 propanone-1

On ajoute en 75 min. à température ambiante, 22,8g ($142.10^{-3}$ mole) de brome à une solution de 20g ($141.10^{-3}$ mole) de propionyl-2 thiophène dans 20 ml de dioxanne anhydre. On laisse l'exothermicité se développer et on agite encore durant 2 heures à température ambiante. On ajoute alors lentement 40ml d'eau contenant de l'hydrosulfite de sodium.

Après extraction à l'éther éthylique, on lave les phases organiques à l'eau salée, on les sèche sur sulfate de sodium et on les amène à sec sous vide. On obtient ainsi 31,10g de bromo-2 (thiényl-2)-1 propanone-1 titrant 89,3% en C.P.G. soit un rendement molaire de 89,9%.

On prépare un échantillon analytique par distillation sous vide.

P.E. : 95°C sous 0,7 mm Hg

C.P.G. : 98,0%

$n_D^{20}$ : 1,6014

I.R. (film) : 3100, 1660 $cm^{-1}$

R.M.N. ($CDCl_3$)

8,00-7,00 (m, 3H) ; 5,10 (q, 1H) ; 1,90 ppm (d, 3H)

Analyse

| | | | | | | |
|---|---|---|---|---|---|---|
| C | calculé | : | 38,37% | trouvé | : | 38,29% |
| H | calculé | : | 3,22% | trouvé | : | 3,22% |
| S | calculé | : | 14,63% | trouvé | : | 13,94% |
| Br | calculé | : | 36,47% | trouvé | : | 36,69% |

b) Acide hydroxy-2 (thiényl-2)-2 propionique

En opérant comme décrit dans l'Exemple 9 à partir de 2g de bromo-2 (thiényl-2)-1 propanone-1, on obtient 1,40g d'acide hydroxy-2 (thiényl-2)-2 propionique titrant 76,8% en C.P.G. soit un rendement molaire de 72,7%.

On prépare un échantillon analytique par chromatographie sur trisilicate de magnésium en utilisant un mélange chlorure de méthylène/méthanol comme éluant et en recristallisant dans le toluène.

P.F. : 113°C

C.P.G. : 98,9%

I.R. (KBr) : 3400, 3300-2500, 1720 $cm^{-1}$

R.M.N. ($CDCl_3/DMSOd_6$)

9,00-6,00 (m, 5H) ; 1,70 ppm (s, 3H)

Analyse

| | | | | | | |
|---|---|---|---|---|---|---|
| C | calculé | : | 48,82% | trouvé | : | 49,07% |
| H | calculé | : | 4,68% | trouvé | : | 4,70% |
| S | calculé | : | 18,62% | trouvé | : | 18,26% |

## EXEMPLE 12

Préparation de l'acide hydroxy-2 phényl-2 propionique

a) Bromo-2 phényl-1 propanone-1

On ajoute 33,6g (0,21 mole) de brome, en 20 min. à 20°C, à une solution de 27,1g (0,2 mole) de phényl-1 propanone-1 dans un mélange de 22ml d'éther éthylique et de 5ml de dioxanne anhydre. On agite encore durant 1h à 20°C puis on ajoute lentement 60ml d'eau sans dépasser 25°C. On extrait à l'éther éthylique puis on lave les phases organiques à l'eau, on les sèche sur sulfate de sodium et on les amène à sec sous vide.

On obtient ainsi 43,25g de bromo-2 phényl-1 propanone-1 titrant 95,1%

en C.P.G. soit un rendement molaire de 96,5%. Après distillation, on obtient un produit dont les caractéristiques sont les suivantes :

P.E. : 92°C sous 0,4 mm Hg

C.P.G. : 96,3%

I.R. (film) : 1690 $cm^{-1}$

R.M.N. ($CDCl_3$)

7,95 et 7,50 (2m, 5H); 5,25 (q, 1H); 1,85 ppm (d, 3H)

b) Acide hydroxy-2 phényl-2 propionique

En opérant comme décrit à l'Exemple 9 à partir de 2g de bromo-2 phényl-1 propanone-1, on obtient 1,12g d'acide hydroxy-2 phényl-2 propionique titrant 58,9% en C.P.G. soit un rendement molaire de 43,9%

On prépare un échantillon analytique après recristallisation dans l'éther isopropylique.

P.F. : 84°C

C.P.G. : 97,6%

I.R. (KBr)

3480, 3280, environ 2900 large ; 1710 $cm^{-1}$

R.M.N. ($DMSOd_6$)

7,80-7,20 (m, 7H); 1,75 ppm(s, 3H)

EXEMPLE 13

Préparation de l'acide (chloro-5 méthoxy-6 naphtyl-2)-2 hydroxy-2 propionique

a) Chloro-2 (chloro-5 méthoxy-6 naphtyl-2)-1 propanone-1

On fait barboter du chlore pendant 2 heures à 90-95°C dans une solution de 21,4g (0,1 mole) de (méthoxy-6 naphtyl-2)-1 propanone-1 (C.P.G. : 100%) dans 100ml de N,N-diméthylformamide anhydre.

On refroidit ensuite le milieu réactionnel à 20°C, on dégaze à l'azote puis on additionne environ 100ml d'eau et 100ml d'éther éthylique. On filtre le produit cristallisé on le lave à l'eau puis à l'éther éthylique et on recristallise dans le méthanol.

Après sèchage, on obtient 12g de chloro-2 (chloro-5 méthoxy-6 naphtyl-2) -1 propanone-1 titrant 96,7% en C.P.G. soit un rendement molaire de 41,0%.

On prépare un échantillon analytique par recristallisation dans le méthan

P.F. : 133°C

C.P.G. : 96,7%

Chlore : calculé : 25,04% trouvé : 25,16%

I.R. (KBr) : 1675, 1620 $cm^{-1}$

R.M.N. ($CDCl_3$)

8,40-7,10 (m, 5H); 5,35 (q, 1H); 3,95 (s, 3H); 1,75 ppm (d, 3H)

Analyse

| | | | | | | |
|---|---|---|---|---|---|---|
| C | calculé | : | 59,39% | trouvé | : | 59,64% |
| H | calculé | : | 4,27% | trouvé | : | 4,23% |
| Cl | calculé | : | 25,04% | trouvé | : | 25,11% |

b) Acide (chloro-5 méthoxy-6 naphtyl-2)-2 hydroxy-2 propionique

En opérant comme décrit dans l'Exemple 9 à partir de 200ml d'eau, 7,2g ($180.10^{-3}$ mole) d'hydroxyde de sodium en pastilles, 2g ($6,83.10^{-3}$ mole) de chloro-2 (chloro-5 méthoxy-6 naphtyl-2)-1 propanone-1 et 40ml de xylène, on obtient 1,51g d'acide (chloro-5 méthoxy-6 naphtyl-2)-2 hydroxy-2 propionique titrant 55% en C.P.G. soit un rendement molaire de 43,4%.

Après cristallisation dans le chloroforme, on obtient un produit dont les caractéristiques sont les suivantes :

P.F. : environ 208°C

C.P.G. : 92,0%

Acidimétrie : 96,4%

I.R. (KBr)

3400, environ 2920 (large), 1715, 1600 $cm^{-1}$

R.M.N. ($CDCl_3$, $DMSOd_6$) :

8,30-7,35 (m, 7H); 4,00 (s, 3H); 1,9 ppm (s, 3H)

+

\+ +

Les Exemples suivants illustrent la préparation d'acides alcanoïques de formule (I') à partir d'α-hydroxy-acides de formule I.

EXEMPLE I

Préparation de l'acide (isobutyl-4 phényl)-2 propionique à partir du composé correspondant de formule I.

a) Acide (isobutyl-4 phényl)-2 propènoïque

On agite à reflux (avec élimination d'eau par azéotropie) pendant 2 heures, une solution de 2,22g ($9,7.10^{-3}$ mole) d'acide (isobutyl-4 phényl)-2 hydroxy-2 propionique (C.P.G. : 96,9%) et 2,22g d'acide p-toluènesulfonique monohydraté dans 90ml de benzène.

Après avoir refroidi à 20°C, on lave le milieu à l'eau salée, sèche sur sulfate de sodium et amène à sec sous vide.

On obtient ainsi 1,98g d'acide (isobutyl-4 phényl)-2 propènoïque titrant 98,8% en C.P.G. soit un rendement molaire de 99,0%.

On prépare un échantillon analytique par recristallisation dans l'hexane.

C.P.G. : 100%

P.F. : 95°C

I.R. (KBr)

3300-2500, 1670, 1615-1605, 840 $cm^{-1}$

R.M.N. ($CDCl_3$)

11,9 (s, 1H) ; 7,2 (m, 4H) ; 6,5 et 5,95 (2s, 2x1H) ; 2,45 (d, 2H) ; 1,8 environ (m, 1H) ; 0,9 ppm (d, 6H).

Analyse

| | | | | |
|---|---|---|---|---|
| C | calculé | : 76,44% | trouvé | : 76,27% |
| H | calculé | : 7,90% | trouvé | : 7,88% |

En opérant comme ci-dessus, à partir de l'acide (isobutyl-4 phényl)-2 hydroxy-2 butanoïque, on obtient l'acide (isobutyl-4 phényl)-2 butène-2 oïque avec un rendement molaire de 91,9%.

On prépare un échantillon analytique par recristallisation dans l'hexane.

C.P.G. : 96,4%

P.F. : 47°C

I.R.

Environ 3000 $cm^{-1}$, 1695 $cm^{-1}$

R.M.N. ($CDCl_3$)

12,3 (s, 1H) ; 7,3-6,95 (m, 4H) ; 6,35 (q, 1H) ; 2,45 (d, 2H) ; 2,1 (d, 2H) ; 1,9 (m, 1H) ; 0,9 ppm (d, 6H).

Analyse

| | | | | |
|---|---|---|---|---|
| C | calculé | : 77,03% | trouvé | : 76,67% |
| H | calculé | : 8,31% | trouvé | : 8,27% |

b) Acide (isobutyl-4 phényl)-2 propionique

On agite sous atmosphère d'hydrogène durant 1,25 heure à 20°C, une suspension de 0,80g ($3,9.10^{-3}$ mole) d'acide (isobutyl-4 phényl)-2 propèn-oïque et de 40mg de charbon palladié à 5% de palladium, dans 16ml d'éthanol.

Après filtration du catalyseur, on amène le milieu réactionnel à sec sous vide.

On obtient ainsi 0,80g d'acide (isobutyl-4 phényl)-2 propionique ou ibuprofène titrant 98,1% en C.P.G. soit un rendement molaire de 97,1%.

P.F. : 78°C

I.R.

Environ 3000, 1710 $cm^{-1}$

R.M.N. ($CDCl_3$)

9,95 (s, 1H) ; 7,1 (m, 4H) ; 3,65 (q, 1H) ; 2,45 (d, 2H) ; 1,95 (m, 1H) ; 1,45 (d, 3H) ; 0,9 ppm (d, 6H).

En opérant comme ci-dessus mais à partir de l'acide (isobutyl-4 phényl)-2 butène-2 oïque, on obtient l'acide (isobutyl-4 phényl)-2 butanoïque ou butibufène avec un rendement de 100%.

I.R.

Environ 3000, 1705 $cm^{-1}$

R.M.N. ($CDCl_3$)

11,25 (s, 1H) ; 7,1 (m, 4H) ; 3,4 (t, 1H) ; 2,45 (d, 2H) ; 2,2-1,5 (m, 3H) ; 0,9 ppm (d+t, 9H).

Analyse

| | | | | | | |
|---|---|---|---|---|---|---|
| C | calculé | : | 76,33% | trouvé | : | 76,28% |
| H | calculé | : | 9,15% | trouvé | : | 9,30% |

## EXEMPLE II

Préparation de l'acide (isobutyl-4 phényl)-2 propionique à partir du composé correspondant de formule I.

On agite sous atmosphère d'hydrogène pendant 29 heures à 20°C, une suspension de 2,22g ($9,7.10^{-3}$ mole) d'acide (isobutyl-4 phényl)-2 hydroxy-2 propionique (C.P.G. : 96,9%), 0,44g de charbon palladié à 5% de palladium dans 20ml d'acide acétique et 2,2ml d'acide sulfurique. On filtre le milieu réactionnel et on ajoute de l'eau. Après extraction à l'éther éthylique, on lave les phases organiques à l'eau, sèche sur sulfate de sodium et amène à sec sous vide.

On obtient ainsi 2,05g d'acide (isobutyl-4 phényl)-2 propionique ou ibuprofène titrant 88% en C.P.G. soit un rendement molaire de 90,4%.

a) En opérant comme ci-dessus mais à partir de l'acide (isobutyl-4 phényl)-2 hydroxy-2 acétique, on obtient l'acide (isobutyl-4 phényl)-2 acétique ou ibufénac avec un rendement molaire de 95,3%.
   On prépare un échantillon analytique par recristallisation dans l'hexane.

C.P.G. : 99,7%

P.F. : 85°C

I.R. (KBr)

3500-2500, 1695 $cm^{-1}$

R.M.N. ($CDCl_3$)

11,95 (s, 1H) ; 7,1 (m, 4H) ; 3,6 (s, 2H) ; 2,45 (d, 2H) ; 1,95 (m, 1H) ; 0,9 ppm (d, 6H).

Analyse

| | | | | |
|---|---|---|---|---|
| C | calculé : | 74,97% | trouvé : | 75,38% |
| H | calculé : | 8,39% | trouvé : | 8,47% |

b) En opérant comme ci-dessus mais à partir de l'acide (isobutyl-4 phényl)-2 hydroxy-2 butanoïque, on obtient l'acide (isobutyl-4 phényl)-2 butanoïque ou butibufène avec un rendement molaire de 83,1%.

EXEMPLE III

Préparation de l'acide (isobutyl-4 phényl)-2 propionique à partir du composé correspondant de formule I.

On agite durant 6 heures à 60°C, une solution de 2,22g ($9,7.10^{-3}$mole) d'acide (isobutyl-4 phényl)-2 hydroxy-2 propionique (C.P.G. : 96,9%) dans 13ml d'acide acétique et 4,4 ml d'acide iodhydrique à 57%. On refroidit le milieu réactionnel à 20°C et on le verse dans une solution de bisulfite de sodium. Après extraction à l'éther éthylique, on lave les phases organiques à l'eau additionnée de chlorure de sodium, on sèche sur sulfate de sodium et on amène à sec sous vide.

On obtient ainsi 2,06g d'acide (isobutyl-4 phényl)-2 propionique titrant 91% en C.P.G., soit un rendement de 93,9%.

EXEMPLE IV

Préparation de l'acide (méthoxy-6 naphtyl-2)-2 propionique.

On agite, durant 3h à 60°C, un mélange de 0,3g ($1,2.10^{-3}$mole) d'acide hydroxy-2 (méthoxy-6 naphtyl-2)-2 propionique, 1,8 ml d'acide acétique et 0,6 ml d'acide iodhydrique à 57%.

On refroidit le milieu réactionnel à 20°C et on le verse dans une solution aqueuse de bisulfite de sodium. On essore le précipité formé, on le lave à l'eau et on le sèche sous vide à 20°C.

On obtient ainsi 0,268g d'acide (méthoxy-6 naphtyl-2)-2 propionique

ou naproxène sous forme DL titrant 97,9% en C.P.G. soit un rendement molaire de 95%.

P.F. : 115,4°C

C.P.G. : 97,9%

I.R. (KBr)

Environ 3000, 1710, 1610 $cm^{-1}$

R.M.N. ($CDCl_3$)

11,40 (s, 1H) ; 7,75-7,05 (m, 6H) ; 3,85 (s, 3H) ; 1,55 ppm (d, 3H).

EXEMPLE V

Préparation de l'acide (méthoxy-6 naphtyl-2)-2 propionique

a) Acide (méthoxy-6 naphtyl-2)-2 propénoïque

On agite au reflux, avec élimination d'eau pendant 4h, un mélange de 1,15g ($4,62.10^{-3}$ mole) d'acide hydroxy-2 (méthoxy-6 naphtyl-2)-2 propionique, 0,120g d'acide p-toluènesulfonique, 0,005g de monométhyl hydroquinone dans 22 ml de benzène. Après avoir refroidi à 20°C, on filtre le milieu réactionnel et on l'amène à sec sous vide.

Après recristallisation dans le benzène, on obtient 0,610g d'acide (méthoxy-6 naphtyl-2)-2 propénoïque titrant 87,8% soit un rendement molaire de 59,1%.

On prépare un échantillon analytique après lavage à l'eau et recristallisation dans un mélange hexane/éther éthylique.

P.F. : 175°C

Titre acidimétrique : 98,7%

I.R. (KBr)

Environ 2950 large, 1685, 1635, 1605 $cm^{-1}$

R.M.N. ($CDCl_3/DMSOd_6$)

7,90-6,90 (m, 7H) ; 6,35 et 5,95 (2s, 2H) ; 3,85 ppm (s, 3H)

Analyse

| | | | | | | |
|---|---|---|---|---|---|---|
| C | calculé | : | 73,67% | trouvé | : | 73,60% |
| H | calculé | : | 5,30% | trouvé | : | 5,34% |

b) Acide (méthoxy-6 naphtyl-2)-2 propionique

En opérant comme décrit dans l'Exemple I b) à partir d'acide (méthoxy-6 naphtyl-2)-2 propénoïque, on obtient l'acide (méthoxy-6 naphtyl-2)-2 propionique ou naproxène sous forme DL.

## EXEMPLE VI

**Préparation de l'acide (méthoxy-6 naphtyl-2)-2 propionique**

On agite au reflux avec élimination d'eau pendant 16h30 min. une solution de 2g ($7,31.10^{-3}$ mole) d'acide hydroxy-2 (méthoxy-6 naphtyl-2)-2 propionique et 0,2g d'acide p-toluènesulfonique dans 50ml de dichloroéthane puis on refroidit à 20°C.

On ajoute 0,1g de charbon palladié à 5% et après les purges habituelles, on agite sous atmosphère d'hydrogène pendant 6 heures à 20°C. On filtre le catalyseur avec précaution et on amène les phases organiques à sec sous vide.

On obtient ainsi l'acide (méthoxy-6 naphtyl-2)-2 propionique brut ou naproxène sous forme DL, avec un rendement de 48,9%.

## EXEMPLE VII

**Préparation de l'acide (thiényl-2)-2 propionique.**

En opérant comme décrit dans l'Exemple IV à partir de 0,60g ($3,3.10^{-3}$ mole) d'acide hydroxy-2 (thiényl-2)-2 propionique, on obtient 0,50g d'acide (thiényl-2)-2 propionique titrant 98,1% en C.P.G. soit un rendement molaire de 95,3%.

On prépare un échantillon analytique par passage sur colonne de trisilicate de magnésium en utilisant un mélange chlorure de méthylène/méthanol comme éluant.

C.P.G. : 98,1%

R.M.N. ($CDCl_3$)

10,10 (s, 1H) ; 7,30-6,80 (m, 3H) ; 4,00 (q, 1H) ; 1,55 ppm (d, 3H).

REVENDICATIONS

1. Procédé de préparation d'α-hydroxy-acides de formule générale :

$$Cy-\overset{\displaystyle OH}{\underset{\displaystyle R}{C}}-CO_2H \qquad I$$

dans laquelle R représente hydrogène ou un radical alkyle inférieur et Cy représente un radical phényle, naphtyle ou hétérocyclique, ce radical comportant éventuellement un ou plusieurs substituants sélectionnés du groupe formé par des radicaux alkyle inférieur, alkényle inférieur, alkynyle inférieur, alkoxy inférieur et par des atomes d'halogène, caractérisé en ce que :
- on traite une cétone α-monohalogénée de formule générale :

$$Cy-\overset{\displaystyle O}{\overset{\|}{C}}-\underset{\displaystyle R}{CH}-X \qquad II$$

dans laquelle R et Cy ont la même signification que précédemment et X représente chlore, brome ou iode en présence d'une solution aqueuse d'un hydroxyde de métal alcalin, d'un solvant organique non polaire sélectionné d'un hydrocarbure aromatique ou alicyclique et d'un excès d'oxygène, accompagné éventuellement d'un gaz inerte, et ce, à une température comprise entre la température d'ébullition du milieu réactionnel à pression atmosphérique et 240°C sous pression,
- puis on acidifie le sel de métal alcalin formé pour obtenir l'acide désiré.

2. Procédé selon la Revendication 1 caractérisé en ce que l'hydroxyde de métal alcalin est l'hydroxyde de lithium, de sodium ou de potassium.
3. Procédé selon la Revendication 1 caractérisé en ce que l'hydrocarbure aromatique est un xylène.
4. Procédé selon la Revendication 1 caractérisé en ce que R représente hydrogène, méthyle ou éthyle.
5. Procédé selon la Revendication 1 caractérisé en ce que Cy représente un radical isobutyl-4 phényle.
6. Procédé selon la Revendication 1 caractérisé en ce que Cy représente

un radical méthoxy-6 naphtyl-2.

7. Procédé selon la Revendication 1 caractérisé en ce que Cy représente un radical thiényl-2.

8. Procédé selon la Revendication 1 caractérisé en ce que l'on utilise :

   0,5 à 40 parties en poids d'hydroxyde de métal alcalin

   5 à 400 parties en volume d'eau

   2 à 40 parties en volume de solvant organique

et ce, pour 1 partie en poids de cétone α-monohalogénée, le traitement ayant lieu à la température d'ébullition du milieu réactionnel à pression atmosphérique.

9. Procédé selon la Revendication 1 caractérisé en ce que l'on utilise :

   0,5 à 10 parties en poids d'hydroxyde de métal alcalin

   5 à 150 parties en volume d'eau

   1 à 30 parties en volume de solvant organique

et ce, pour 1 partie en poids de cétone α-monohalogénée, le traitement ayant lieu à une température comprise entre la température d'ébullition du milieu réactionnel à pression atmosphérique et 240°C sous pression.

10. Procédé selon la Revendication 1 caractérisé en ce que le traitement a lieu à une température de 180 à 220°C.

11. Procédé selon la Revendication 1 caractérisé en ce que le traitement a lieu en autoclave à une température comprise entre la température d'ébullition du milieu réactionnel à pression atmosphérique et 240°C, par introduction en continu de la cétone α-monohalogénée dans ledit milieu réactionnel.

12. Procédé selon la Revendication 1 caractérisé en ce que l'oxygène accompagné d'un gaz inerte représente l'air.

13. Dérivés d'α-hydroxy-acides de formule générale :

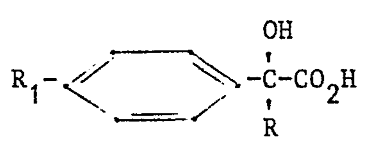

dans laquelle R représente hydrogène ou un radical alkyle ayant de 1 à 4 atomes de carbone et $R_1$ représente un radical alkyle ayant de 1 à 4 atomes de carbone, obtenus en application du procédé selon l'une quelconque des Revendications 1 à 12.

Office européen des brevets

# RAPPORT DE RECHERCHE EUROPEENNE

0184573

Numéro de la demande

EP 85 87 0166

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | EP-A-0 071 299 (BLASCHIM)<br>* Revendications 1,2 *<br>----- | 1 | C 07 C 59/48<br>C 07 C 59/64<br>C 07 C 51/00<br>C 07 D 333/38 |
| | | | **DOMAINES TECHNIQUES RECHERCHES (Int Cl 4)** |
| | | | C 07 C 59/00<br>C 07 C 51/00<br>C 07 C 67/00<br>C 07 C 69/00 |

Le présent rapport de recherche a été établi pour toutes les revendications

| Lieu de la recherche | Date d achevement de la recherche | Examinateur |
|---|---|---|
| LA HAYE | 03-03-1986 | KLAG M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant